# EUROPÄISCHE PATENTSCHRIFT

(11) **EP 0 363 869 B1**
(45) Veröffentlichungstag und Bekanntmachung des Hinweises auf die Patenterteilung: **19.01.1994**
(21) Anmeldenummer: 89118709.8
(22) Anmeldetag: 09.10.1989
(51) Int. Cl.: C07D 333/20

(54) **Substituierte thienylethylamine und Verfahren zu deren Herstellung**
Substituted thienyl ethyl amines and process for their preparation
Thiényléthylamines substituées et procédé de préparation

(30) Priorität: 11.10.1988 CH 3795/88
(43) Veröffentlichungstag der Anmeldung: 18.04.1990
(73) Patentinhaber: LONZA AG, CH-3945 Gampel/Wallis (CH)
(72) Erfinder: Warm, Aleksander, Dr., Visp (Kanton Wallis) (CH); McGarrity, John, Dr., Visp (Kanton Wallis) (CH)
(74) Vertreter: Weinhold, Peter, Dr.

(56) Entgegenhaltungen:
- EP-A- 0 262 395
- US-A- 4 752 613
- JOURNAL OF HETEROCYCLIC CHEMISTRY, Band 22, Nr. 4, Juli-August 1985, Seiten 1011-1016, Hetero Corp.; D. FREHEL et al.: "New synthesis of 5,6,7,8-tetrahydro-4eta-thieno[2,3-delta]azepine"

## Beschreibung

Die Erfindung betrifft substituierte Thienylethylamine der allgemeinen Formel 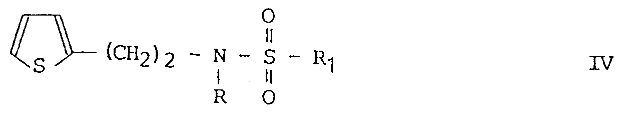
worin R Formyl, Acetyl oder Benzoyl bedeutet oder Benzyl ist, das wenigstens durch ein Halogenatom ringsubstituiert ist, und R₁ einen Alkylrest mit 1-6, insbesondere 1-4 Kohlenstoffatomen, Phenyl oder Methylphenyl bedeutet, sowie ein Verfahren zur Herstellung der neuen Verbindungen.

Die genannten substituierten Thienylethylamine können als Zwischenprodukte für die Herstellung von anti-thrombolytisch wirksamen 4,5,6,7-Tetrahydro-thieno-[3,2c]-pyridinen eingesetzt werden (E. Saltiel et al, Drugs, 34 (1987), S.222).

Die US-A-4.752.613 beschreibt Sulfonamidothienylcarbonsäuren, die als Thromboxanrezeptor-Antagonisten verwendet werden können. Als Zwischenprodukte auf dem Weg zu diesen Säuren werden Verbindungen der Formel 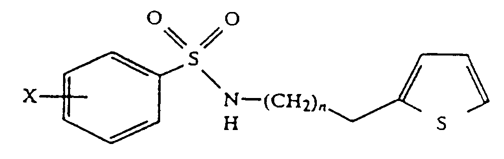
beschrieben, worin X Halogen, Niederalkyl, Arylalkyl, Alkoxy oder Hydroxy bedeutet und n eine ganze Zahl von 0 bis 3 ist, zu deren Herstellung eine Suspension aus einem Amin der Formel 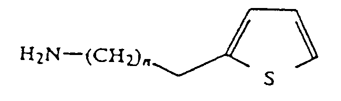
worin n die obige Bedeutung hat, und aus wasserfreiem Natriumacetat in Ethanol mit einem entsprechenden Arylsulfonylchlorid in Ethanol umgesetzt wird.

Fréhel, D. et al. (J. Heterocycl. Chem. 22, 1011-1016; 1985) beschreiben ein Verfahren zur Herstellung von Ticlopidinderivaten, das über 4-Methyl-N-[2-(2-thienyl)ethyl]-benzolsulfonamid als Zwischenstufe verläuft. Diese Zwischenstufe wird durch Umsetzung von Thienylethylamin mit Tosylchlorid erhalten und anschließend in einem mehrstufigen Verfahren unter Substitution des Tosylrests in die gewünschten Ticlopidinderivate überführt.

Die substituierten Ethanolamine der allgemeinen Formel 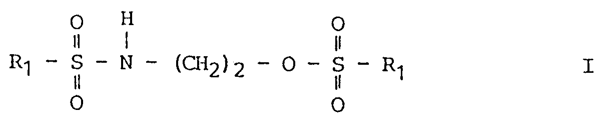
als Ausgangsprodukte des erfindungsgemässen Verfahrens sind einfach zuganglich aus Ethanolamin durch dessen Umsetzung mit zwei Aequivalenten des entsprechenden Halogenids 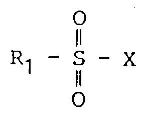
worin R₁ die genannte Bedeutung hat und X Chlor, Brom oder Jod bedeutet.

Die Ueberführung des substituierten Ethanolamins I in das Aziridin II der allgemeinen Formel 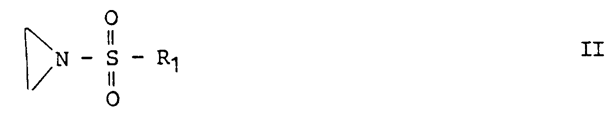
gelingt mit einer starken Base in Gegenwart eines inerten Lösungsmittels.

Als Base können die in der Fachwelt üblichen und Vertreter, zweckmässig Alkalihydride, Alkaliamide oder Alkyllithium, verwendet werden. Vorzugsweise setzt man Alkalihydride, wie z.B. Natriumhydrid, ein.

Als inerte Lösungsmittel werden zweckmässig Ether, wie z.B. Tetrahydrofuran, Diethylether, Dimethoxyethan oder Butylmethylether, verwendet.

Die Umsetzungstemperatur bewegt sich vorteilhaft zwischen -80 und +100°C, besonders vorteilhaft zwischen 0 und +20°C.

Das resultierende Aziridin kann isoliert werden, vorzugsweise wird es aber direkt mit 2-Thienyllithium in das entsprechende Thienylethylamin der Formel 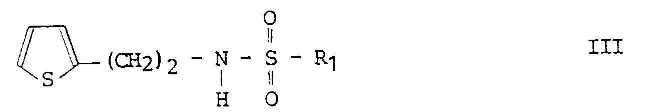 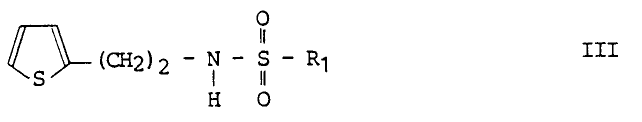
worin R₁ die genannte Bedeutung hat, überführt.

Das 2-Thienyllithium kann auf bekannte Weise, Z.B. gemäss Chadwick et al, J.Chem.Soc. Perkin I, 1977, S.887, aus Thiophen und z.B. Butyllithium in einem geeigneten Lösungsmittel, vorzugsweise gleichzeitig und parallel zur ersten Stufe des erfindungsgemässen Verfahrens, generiert und der zweiten Stufe zugesetzt werden.

Die Umsetzung mit 2-Thienyllithium erfolgt vorteilhaft im gleichen Lösungsmittel wie die erste Stufe bei Temperaturen zwischen -80 und +60°C.

Das resultierende Thienylethylamin der Formel worin R₁ die genannte Bedeutung hat, wird vorteilhaft wiederum nicht isoliert, sondern direkt mit dem Reaktanden der Folgestufe, Verbindungen der Formel RX, versetzt. In diesen Verbindungen hat R die genannte Bedeutung, während X für ein Chlor-, Brom- oder Jodatom steht.

Geeignete Vertreter der Verbindungen RX sind beispielsweise Acetylchlorid, Benzoylchlorid, Benzylchlorid oder o-Chlorbenzylchlorid.

Als Lösungsmittel wird vorteilhaft dasjenige der Vorstufe übernommen. Es ist aber auch möglich, das Lösungsmittel durch ein polares Lösungsmittel zu ersetzen.

Zweckmässig arbeitet man bei Reaktionstemperaturen zwischen -50 und +100°C.
Ist R in der Verbindung RX Benzyl oder o-Chlorbenzyl, liegt die Reaktionstemperatur vorzugsweise zwischen +20 und +100°C, bei R = Acetyl oder Benzoyl vorzugsweise zwischen -10 und +20°C.

Das Zielprodukt kann nach beendeter Umsetzung auf übliche Weise isoliert und gereinigt werden.

### Beispiel

### Herstellung von N-[(2-Chlorphenyl)methyl]-4-methyl-N-[2-(2-thienyl)ethyl]-benzolsulfonamid

a) Herstellung von N-[(4-Methylphenyl)sulfonyl]-aziridin
   118 mg (3 mmol) Natriumhydrid (55% in Oel) wurden in 3 ml Tetrahydrofuran vorgelegt und auf 0°C abgekühlt. Während 7 min gab man 1 g (2,7 mmol) N,O-Bis-(toluolsulfonyl)-ethanolamin in 2 ml Tetrahydrofuran zu (Gasentwicklung). Das Reaktionsgemisch wurde auf Raumtemperatur erwärmt. Dann wurden 2 ml Ether zugegeben und während 50 min bei Raumtemperatur gerührt.
b) Herstellung von 2-Thienyllithium
   228 mg (27 mmol) Thiophen und 315 mg (2,7 mmol) Tetramethylethylendiamin wurden in 3 ml Ether bei Raumtemperatur vorgelegt. Dann gab man 1,1 ml (2,7 mmol) Butyllithium (2,5 M in Hexan) innert 2 min zu (exotherm). Man liess 30 min bei Raumtemperatur nachrühren.
c) Herstellung von 4-Methyl-N-[2-(2-thienyl)ethyl]-benzolsulfonamid
   Das Reaktionsgemisch aus Stufe b) wurde nun bei Raumtemperatur innert 3 min zum Reaktionsgemisch aus Stufe a) gegeben. Dann wurden 3 ml Tetrahydrofuran zugegeben, um die Suspension besser rührbar zu machen. Man liess 40 min bei Raumtemperatur rühren.
d) Herstellung von N-[(2-Chlorphenyl)methyl]-4-methyl-N-[2-(2-thienyl)ethyl]-benzolsulfonamid
   Dem Reaktionsgemisch aus Stufe c) wurden 654 mg (4,05 mmol) 2-Chlorbenzylchlorid hinzugegeben. Das Reaktionsgemisch wurde dann zum Rückfluss erhitzt. Man liess 4 h bei Rückflusstemperatur (62°C) rühren. Nach Abkühlen auf Raumtemperatur wurden 20 ml Wasser zugegeben. Das Tetrahydrofuran wurde im Vakuum entfernt. Der wässrige Rückstand wurde 3 mal mit je 15 ml Methylenchlorid extrahiert. Die organische Phase wurde abgetrennt, mit Magnesiumsulfat getrocknet und schliesslich eingedampft. Dabei resultierte ein dunkles Oel, woraus das Produkt auskristallisierte.

- Ausbeute:: 860 mg N-[(2-Chlorphenyl)methyl]-4-methyl-N-[2-(2-thienyl)ethyl]-benzolsulfonamid = 78%.
- Fp :: 95,5-96,5°C.
- ¹H-NMR:: (CDCl₃, 300 MHz) δ in ppm
7,75, d, J = 8,5 Hz, 2H
7,55, dd, J = 7 Hz, 2,5 Hz, 1H
7,37-7,31, m, 3H
7,30-7,20, m, 2H
7,08, d, J = 5 Hz, 1H
6,87, dd, J = 5 Hz, 3,5 Hz, 1H
6,68, d, J = 3,5 Hz, 1H
4,51, s, 2H
3,40, dd, J = 8 Hz, 8 Hz, 2H
2,88, dd, J = 8 Hz, 8 Hz, 2H
2,45, s, 3H

## Patentansprüche

1. Substituierte Thienylethylamine der allgemeinen Formel 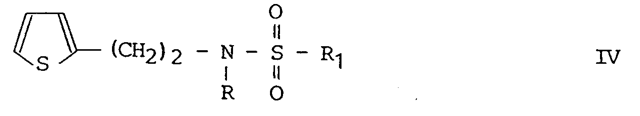 worin R Formyl, Acetyl oder Benzoyl bedeutet oder Benzyl ist, das wenigstens durch ein Halogenatom ringsubstituiert ist, und R₁ einen Alkylrest mit 1-6 Kohlenstoffatomen, Phenyl oder Methylphenyl bedeutet.

2. Verfahren zur Herstellung von Thienylethylaminen der allgemeinen Formel nach Anspruch 1, dadurch gekennzeichnet, dass man ein substituiertes Ethanolamin der allgemeinen Formel 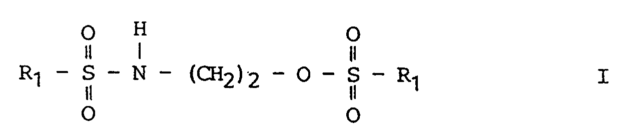 worin R₁ die genannte Bedeutung hat, mit einer starken Base in ein Aziridin der Formel 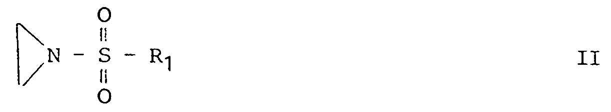 worin R₁ die genannte Bedeutung hat, überführt, mit 2-Thienyllithium in ein Thienylethylamin der Formel 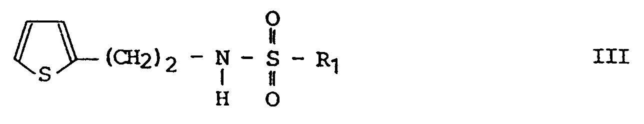 worin R₁ die genannte Bedeutung hat, umwandelt und schliesslich mit einer Verbindung RX, worin R die genannte Bedeutung hat, und X Chlor, Brom oder Jod bedeutet, zum Endprodukt umsetzt.

3. Verfahren nach Patentanspruch 2, dadurch gekennzeichnet, dass als starke Base ein Alkalihydrid, ein Alkaliamid oder ein Alkyllithium verwendet wird.

4. Verfahren nach mindestens einem der Patentansprüche 2-3, dadurch gekennzeichnet, dass die Umsetzung in das Aziridin bei Temperaturen zwischen -80 und +100°C in einem inerten Lösungsmittel erfolgt.

5. Verfahren nach mindestens einem der Patentansprüche 2-4, dadurch gekennzeichnet, dass die Umsetzung mit 2-Thienyllithium bei Temperaturen zwischen -80 und +60°C in einem inerten Lösungsmittel erfolgt.

6. Verfahren nach mindestens einem der Patentansprüche 2-5, dadurch gekennzeichnet, dass die Umsetzung mit einer Verbindung RX bei Temperaturen zwischen -50 und +100°C erfolgt.

7. Verfahren nach mindestens einem der Patentansprüche 2-6, dadurch gekennzeichnet, dass ohne Isolierung der Zwischenprodukte verfahren wird.

## Claims

1. Substituted thienyl ethyl amines of the general formula 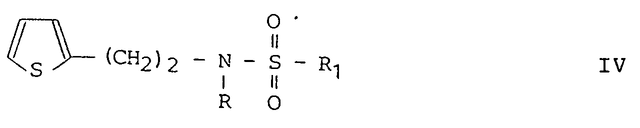 wherein R is formyl, acetyl or benzoyl, or benzyl ring-substituted by at least one halogen atom, and R₁ is alkyl having 1 to 6 carbon atoms, phenyl or methylphenyl.

2. A process for preparing thienyl ethyl amines of the general formula set forth in Claim 1, characterized in that a substituted ethanol amine of the general formula 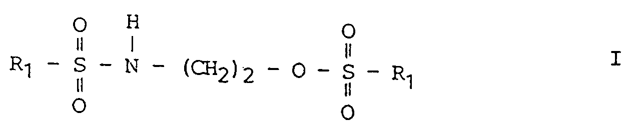 wherein R₁ has the same meaning as above; is converted with a strong base into an aziridine of the formula 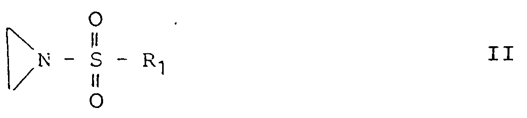 wherein R₁ has the same meaning as above; this compound is converted with 2-thienyl lithium to a thienyl ethyl amine of the formula 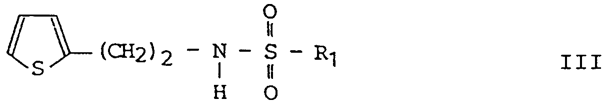 wherein R₁ has the same meaning as above; and which is finally reacted with a compound of formula RX wherein R has the same meaning as above and X is chlorine, bromine or iodine, to form the final product.

3. The process according to Claim 2, characterized in that as a strong base an alkali hydride, an alkali amide, or an alkali lithium is used.

4. The process according to at least one of Claims 2 and 3, characterized in that the reaction to the aziridine takes place at temperatures between -80 and +100°C in an inert solvent.

5. The process according to at least one of Claims 2 to 4, characterized in that the reaction with 2-thienyl lithium takes place at temperatures between -80 and +60°C in an inert solvent.

6. The process according to at least one of Claims 2 to 5, characterized in that the reaction with a compound of formula RX takes place at temperatures between -50 and +100°C.

7. The process according to at least one of Claims 2 to 6, characterized in that no isolation of the intermediate products is carried out.

## Revendications

1. Thiényléthylamines substituées de la formule générale 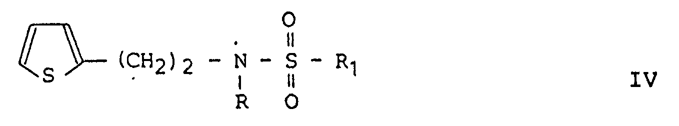 dans laquelle R signifie formyle, acétyle ou benzoyle ou est un benzyle qui est substitué cycliquement au moins par un atome d'halogène, et R₁ signifie un reste alkyle avec 1-6 atomes de carbone, phényle ou méthylphényle.

2. Procédé pour la préparation de thiényléthylamines de la formule générale selon la revendication 1, caractérisé en ce que l'on transforme une éthanolamine substituée de la formule générale 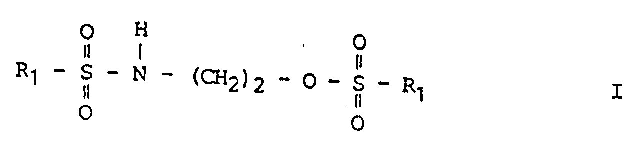 dans laquelle R₁ a la signification citée, avec une base forte, en aziridine de formule 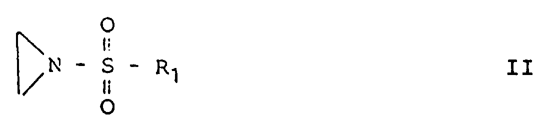 dans laquelle R₁ a la signification citée, que l'on convertit avec 2-thienyllithium- en une thiénylethylamine de formule 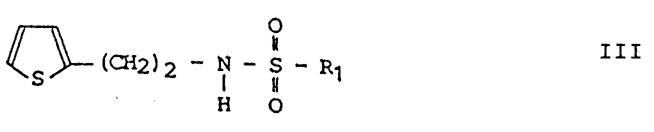 dans laquelle R₁ a la signification citée, et que l'on fait réagir pour finir avec un composé RX, dans lequel R a la signification citée, et X signifie le chlore, le brome ou l'iode, pour obtenir le produit final.

3. Procédé selon la revendication 2, caractérisé en ce qu'en tant que base forte on utilise un hydrure alcalin, un amide alcalin ou un alkyllithium.

4. Procédé selon au moins selon l'une des revendications 2, 3, caractérisé en ce que la transformation en l'aziridine s'effectue à des températures entre -80 et + 100°C dans un solvant inerte.

5. Procédé selon au moins l'une des revendications 2 à 4, caractérisé en ce que la réaction avec le 2-thiényllithium s'effectue à des températures entre -80 et +60°C dans un solvant inerte.

6. Procédé selon au moins l'une des revendications 2 à 5, caractérisé en ce que la réaction s'effectue avec un composé RX à des températures entre -50 à +100°C.

7. Procédé selon au moins l'une des revendications 2 à 6, caractérisé en ce que l'on procède sans isoler les produits intermédiaires.
